# EUROPEAN PATENT APPLICATION

(11) **EP 3 663 762 A1**
(43) Date of publication of application: **10.06.2020**
(21) Application number: 18306605.9
(22) Date of filing: 03.12.2018
(51) Int. Cl.: G01N 33/68

(54) **INTERLEUKIN-6 AS DISEASE SEVERITY BIOMARKER IN FACIOSCAPULOHUMERAL MUSCULAR DYSTROPHY TYPE 1 (FSHD1)**

(71) Applicant: Centre Hospitalier Universitaire de Nice, 06200 Nice (FR); Université de Nice Sophia Antipolis, 06100 Nice (FR); Centre National de la Recherche Scientifique - CNRS, 75016 Paris (FR); INSERM (Institut National de la Santé et de la Recherche Médicale), 75013 Paris (FR)
(72) Inventor: SACCONI, SABRINA, 06000 NICE (FR); GLAICHENHAUS, Nicolas, 06100 NICE (FR)
(74) Representative: Plasseraud IP

(57) **Abstract**

The invention relates to Interleukin-6 (IL-6) as a biomarker of the severity and rate of disease progression of Facioscapulohumeral muscular dystrophy type I (FSHD1) in humans and a therapeutic target to treat FSDH1.

## Description

### Field of the invention

The invention relates to Interleukin-6 (IL-6) as a biomarker of the severity and/or the rate of disease progression of Facioscapulohumeral muscular dystrophy type I (hereinafter FSHD1) in humans and a therapeutic target to treat FSDH1.

### Background of the invention

Facioscapulohumeral muscular dystrophy type I (hereinafter FSHD1) is the third most common degenerative myopathy in adults affecting males and females of all ages. Typical clinical phenotype and pattern of muscle wasting characterize this autosomal-dominant inherited disease, as well as an important inter and intra-familial variability of clinical expression.

FSHD1 has been associated to pathogenic contraction of D4Z4 repeated units occurring in a permissive chromosome 4 and causing epigenetic alterations in D4Z4 macrosatellite leading to an inappropriate expression of stable DUX4 transcript in muscle (Lemmers et al., 2010). DUX4p is strongly cytotoxic, especially in myoblasts. It initiates a large transcription deregulation cascade leading to muscle atrophy and oxidative stress. The DUX4 downstream pathway leading to cell death is not well understood.

A few studies have suggested that DUX4 cytotoxicity was associated with innate and adaptive immunity deregulation (Geng et al., 2012; Tasca et al., 2018). Inflammatory pathways induced by DUX4 inappropriate expression may accelerate and/or participate to progression of muscle degeneration. The presence of inflammation in FSHD1 patient's muscles has been observed in several studies. Nevertheless, two pathological patterns seem to emerge at histological and immune-histochemical analysis of differentially affected muscles. Some authors have described inflammatory macrophages in FSHD1 patients exhibiting characteristic dystrophic changes (increase in connective tissue, fiber size variation, necrotic and regenerative fibers) (Olsen et al., 2006). In another study, infiltrating T lymphocytes in endomysium and perivascular area have been observed (Frisullo et al., 2011). These two descriptions may correspond to different stages of muscle inflammation, an early stage characterized by more acute changes and a later stage associated with a more chronic damage. It has been suggested that in FSHD1 patients, muscle inflammation seems to be involved in early stages of disease progression and may not necessarily results in muscle degeneration. As other genetic defined muscular dystrophies, like Duchenne Dystrophy or dysferlinopathies, cellular damage may lead to toxic molecules release (DNA, RNA, DAMPs and PAMPS, etc...) in extracellular space. In chronic inflammatory state, dead muscle fibers may not be properly removed, and the toxic contents may leak out of the cell and worsen the damage surrounding cells and tissues accelerating muscle degeneration and progressive fibrosis. In support of this hypothesis, muscle MRI studies have shown that muscle edema/inflammation (i.e. muscle showing T2-STIR hyper-intense signal at MRI) always precede muscle fibro-fatty degeneration (i.e. muscle showing T1 hyperintense signal at MRI) but T2-STIR hyper-intense muscles do not necessarily become fibro-fatty infiltrated, suggesting that in early stages of the disease when muscle inflammation is not "chronic", the progression may be interrupted. Little is known concerning the specific type of immunity deregulation implicated in FSHD1 progression, although some evidences exist that drugs, like corticosteroids, inducing a non-specific dampening of the acute and chronic inflammatory response are not effective in preventing disease evolution (Tawil et al., 1997). Nevertheless, in analogy to what is seen for other "inflammatory" diseases showing chronic inflammatory deregulation, more specific therapeutic approaches may be beneficial.

There is currently no pharmacological treatment available for FSHD1. Clinical trials with novel therapeutics have been discouraged both by the lack of a recognized mouse model and by the fact that FSHD1 is a highly variable and slowly progressive disease, whereas the efficacy of therapeutic interventions is ideally established over short periods of time. Therefore, there is a need for a method that could be used to assay responsiveness to therapy in order to facilitate FSHD1 therapeutic development and clinical research.

A few studies have aimed at identifying biomarkers related to disease activity in FSHD1. For example, Turki et al. have found that about functional muscle impairment in FSHD1 correlated with oxidative stress and mitochondrial dysfunction (Turki et al., 2012). In another study, Statland et al., performed a prospective cross-sectional study of serum biomarkers in 22 FSHD patients (19 FSHD1, 3 FSHD2) compared to 23 age and gender-matched healthy controls using a commercial multiplex, microsphere-based immune-fluorescent assay of 243 markers. 169 markers had values sufficient for analysis. Correction for multiple testing identified 7 biomarkers below a 5% false discovery rate: creatine kinase MB fraction (CKMB), tissue-type plasminogen activator (PLAT), myoglobin, epidermal growth factor (EGF), chemokine (C-C motif) ligand 2 (CCL2), CD40 ligand (CD40L) (Statland et al., 2015) and vitronectin (VTN). Moderate correlations to measures of FSHD disease were seen for CKMB, PLAT, and EGF. Markers in the plasminogen pathway (PLAT, serpin peptidase inhibitor, and VTN) were correlated with each other in FSHD but not healthy controls.

**Summary of the invention:** Although FSDH1 is a genetic disease, the inventors proposed that the immune system plays a decisive role in the progression of the disease, notably by promoting the destruction of muscle cells.

In order to test this hypothesis, the inventors studied the association between the concentrations of 14 cytokines and four clinical scores that are currently used to assess disease severity: The Clinical Severity Score (CSS), the Manual Muscular Testing (MMT) sum score, the Brooke score and the Vignos score. The inventors observed that Interleukin-6 (hereinafter IL-6) serum concentration was inversely correlated directly correlated with the CSS, the Brooke score and the Vignos score, and inversely correlated with the MMT sum score, preferably in men and menopausal women, most preferably in men.

Thus, the inventors discovered that Interleukin-6 (IL-6) is an excellent biomarker of the severity and rate of disease progression of FSDH1 in humans and possibly a therapeutic target to treat FSDH1.

### Brief description of the drawings

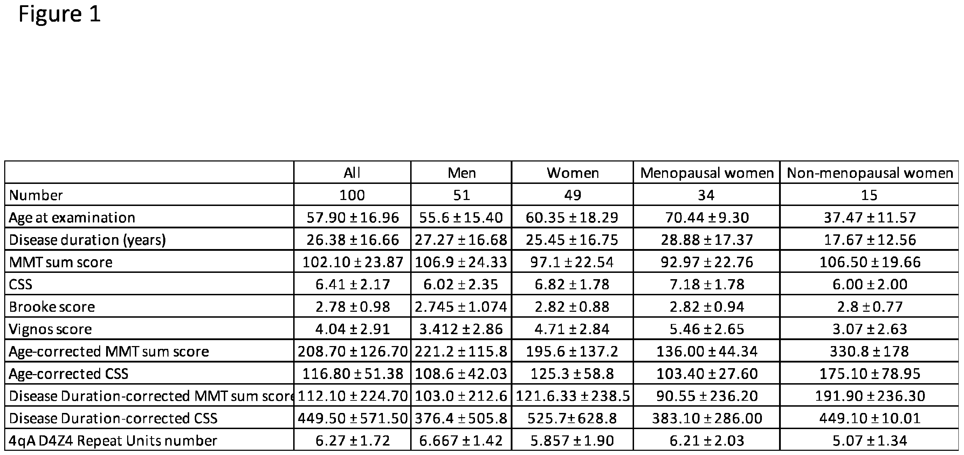

**Figure 1****: Patient demographic and clinical characteristics**
   The number of patients, age at examination (years), disease duration (years), lower and upper limbs Manual Muscle Testing (MMT sum score), Clinical Severity Score (CSS), Brooke score and Vignos score and 4QA D4Z4 number of repeated units are indicated for all patients, men, women, menopausal women and non-menopausal women.
**Figure 2****: Correlations between cytokines and clinical scores**
   Spearman's correlation coefficient (R) and False Discovery Rate (FDR) for the indicated cytokines and clinical scores in all patients, men and women.
**Figure 3****: Heat maps showing correlations between serum biomarker levels and clinical scores.**
   Heat maps of Spearman's correlation coefficient (left panel) and False Discovery Rate (FDR) (right panel) in all patients **(A)**, men **(B)** and women **(C).**
**Figure 4****: Association between serum levels of IL-6 and CSS and age-adjusted CSS**
   Scatter plot representations of serum IL-6 levels versus CSS (A) and age-adjusted CSS (B) in all patients, men, women, and menopausal and non-menopausal women. ****, p < 0.0001; ***, p < 0.001; **, p < 0.01. R coefficients are indicated.
**Figure 5****: Association between serum levels of IL-6 and MMT sum score, Brooke score and Vignos score**
   Scatter plot representations of serum IL-6 levels versus MMT sum score **(A)**, Brooke score **(B)** and Vignos score **(C)** in all patients, men, women, and menopausal and non-menopausal women. **, p < 0.01; *, p < 0.05.
**Figure 6****: Comparison of serum levels of IL-6 between different patient groups**
   **(A)** All patients were stratified into four groups based on their gender and menopausal status for women.
   **(B)** All patients, men, women and menopausal and non-menopausal women were stratified into those with CSS > 6 and those with a CSS ≤ 6. *, p < 0.05; **, p < 0.01; **.
**Figure 7****: Association between serum levels of IL-6 and patient age**
   Scatter plot representations of serum IL-6 levels versus age in all patients, men, women, and menopausal and non-menopausal women as well as in healthy individuals. ****, p < 0.0001; ***, p < 0.001.

### Detailed description of the invention

In a first aspect, the invention relates to an *in vitro* method for assessing the disease severity and/or the rate of disease progression in a patient suffering from Facioscapulohumeral muscular dystrophy, comprising the step of measuring the concentration of IL-6 in a biological sample of said patient and wherein the concentration of IL-6 is positively correlated with the disease severity.

As used herein, the term "assessing" refers to a method that allows determining with a high level of probability (statistically significant) the disease severity.

As used herein, the term "biological sample" is intended to include tissues, cells, biological fluids and isolates thereof, isolated from a patient, as well as tissues, cells and fluids present within a patient, provided that said biological sample is susceptible to contain (i) peripheral blood cells or (ii) nucleic acids or proteins that are produced by the peripheral blood cells from the patient. In a specific embodiment, said biological sample for use in the methods of the invention is a blood sample, including, peripheral blood cell sample and proteins.

Preferably, the biological sample is a serum sample or a plasma sample of said patient, most preferably a serum sample.

The term "patient" refers to a human being suffering from Facioscapulohumeral muscular dystrophy.

Said patient may be a man or a menopausal woman, preferably a man. Also, said patient is preferably an adult.

Said method for assessing the disease severity and/or the rate of disease progression may be followed by a step of providing the patient with an appropriate treatment, preferably with an inhibiting agent of the expression or the function of IL-6 such as Short interfering RNA (siRNA) or anti-IL-6 antibodies, preferably anti-IL-6 antibodies or with an inhibiting agent of the action of the IL-6, such as anti-CD 126-antibodies (antibodies targeting the interleukin 6 receptor).

As used herein the term "antibody" or "immunoglobulin" includes whole antibodies and any antigen binding fragments or derivatives (i.e., "antigen-binding portion") or single chains thereof. In a specific embodiment, such antibody or immunoglobulin may include monoclonal or polyclonal antibodies or immunoglobulins, or immunoglobulins obtained from immunized animals or from recombinant cells, and their antigen-binding portions.

As used herein, and as well understood in the art, "treatment" is an approach for obtaining beneficial or desired results, including clinical results. Beneficial or desired results can include but is not limited to, alleviation or amelioration of one or more symptoms or conditions, diminishment of extent of disease, stabilized (i.e. not worsening) state of disease, preventing spread of disease, delay or slowing of disease progression, reversal of disease, amelioration or palliation of the disease state, and remission (whether partial or total).

The "disease severity" can typically be assessed by four clinical scores: the Clinical Severity Score (CSS), the Manual Muscular Testing Score (MMT) (Kilmer et al., 1995), the Brooke score (Brooke et al., 1981) and the Vignos scores (Vignos et al., 1963). CSS can be adjusted or not by age at clinical assessment.

MMT is a procedure for the evaluation of the function and strength of 28 individual upper and lower limbs muscles or muscle groups based on the effective performance of a movement in relation to the forces of gravity and manual resistance. The MMT score ranges from 0 (less severe) to 140 (more severe cases).

CSS is a 10-grade score that considers the extent of weakness in various body regions and considers the descending spread of symptoms from face and shoulders to pelvic and leg muscles typical of FSHD1 (Ricci et al., 1999). The CSS scale ranges from 1 (less severe) to 10 (more severe cases).

The Vignos score considers functional activities such as walking, going up and down stairs, sitting and standing from the chair and from the floor as essential in classifying considers the stages of patients with DMD (Vignos et al., 1963). Vignos scale ranges from 1 (less severe) to 10 (more severe cases).

The Brooke score assesses subjectively the upper limb function (Brooke et al., 1981). It involves measuring the timed performance of six tasks, which require more proximal than distal control.

In the clinical and research realms, the Brooke and Vignos functional scales are the most frequently used outcome measures for muscular dystrophy, although initially developed for use with Duchenne muscular dystrophic patients (Brooke et al., 1981; Vignos et al., 1963). Although the scope of both scales is restricted to assessing levels of function for the upper and lower extremities only, they are simple to administer and score, and the reliability and validity have been well documented (Florence et al., 1984)

These clinical scores allow the skilled person to assess the disease severity of FSHD1 in a patient. However, these tests are long and tedious to undertake, they require the patient to be on the premises and the tests have to be performed by a trained person. The present invention provides an easier method to assess the disease severity and the rate of disease progression of FSHD1 that can be used in addition or instead of the MMT, CSS tests, Brooke test and Vignos test.

The inventors showed that the concentration of IL-6 in a biological sample of a patient (preferably men or menopausal women, most preferably men) suffering from FSHD1 is inversely correlated with the MMT score and directly correlated with the CSS score, the Vignos score and the Brooke score. In other words, when the IL-6 concentration in the blood increases, the MMT score decreases and the CSS score increases. Therefore, the concentration of IL-6 in the blood of the patient is positively correlated with the disease severity and the rate of disease progression.

As such, the present invention provides a novel molecular biomarker of FSHD1 that could be used to assess responsiveness to therapy and would greatly facilitate FSHD1 therapeutic development and clinical research and/or to easily provide the patients with specific treatment according to their disease severity and/or rate of disease progression.

The inventors observed that IL-6 concentration was inversely correlated with MMT and directly correlated with CSS. As such, an increased IL-6 concentration in the blood of the patient compared to a corresponding control value is positively correlated with the disease severity and/or the rate of disease progression in said patient.

The control value may be a value obtained my measurement of the IL-6 concentration in a biological sample from the patient at an earlier time point or a reference control value. Typpically a reference control value can be a mean value obtained from a mean population of healthy subjects (i.e.: subjects who are not suffering from FSHD1, preferably who are not suffering from any muscular dystrophy).

The method for assessing the disease severity and/or the rate of disease progression in a patient suffering from Facioscapulohumeral muscular dystrophy as per the present invention typically include the steps of (a) measuring the concentration of IL-6 in a biological sample from said patient to obtain concentration value(s), and (b) comparing the obtained concentration value of said IL-6 biomarker to corresponding control values, wherein the difference in the concentration value(s) compared to the respective control value is indicative of the disease severity and/or the rate of disease progression.

As used herein, the term "decrease" or "increase" means a statistically significant decrease or increase of a control value, preferably, at least 10%, at least 20%, at least 30%, at least 40%, at least 50%, at least 90%, or at least 99% decrease or increase of the control value.

The quantification may be relative (by comparing the amount of the biomarker to a control with known amount of biomarker for example and detecting "higher" or "lower" amount compared to that control) or more precise (i.e.: quantitative), at least to determine the specific amount relative to a known control amount (i.e.: to determine the difference between the concentration value and the control value).

These quantification assays of a biomarker can be conducted in a variety of ways. Appropriate conditions to the particular assay and components thereof will be well known to one skilled in the art.

Various methods are known in the art for quantifying protein levels in biological samples, including immunoassays. They include but are not limited to radioimmunoassays, ELISA (enzyme linked immunosorbent assays), "sandwich" immunoassays, immunoradiometric assays, in situ immunoassays (using e.g., colloidal gold, enzyme or radioisotope labels), western blot analysis, immunoprecipitation assays, immunofluorescent assays, flow cytometry, immunohistochemistry, confocal microscopy, enzymatic assays and surface plasmon resonance).

In a second aspect, the invention relates to a method of monitoring a treatment efficacy in a patient suffering from Facioscapulohumeral muscular dystrophy receiving said treatment by using IL-6 as a biomarker.

More precisely, the present invention relates to an *in vitro*, *ex vivo* or *in vivo* method of monitoring treatment efficacy in a patient suffering from Facioscapulohumeral muscular dystrophy. The method typically comprises determining in a biological sample of the patient at two or more time points the IL-6 protein level of expression (or 11-6 concentration), wherein measurement of a higher IL-6 protein level of expression (typically assessed by determining 11-6 concentration in said biological sample) in a biological sample of the patient at a later time point, compared to a value obtained in a biological sample of the patient at an earlier time point (thus typically used as a reference value), is indicative that the patient is non-responder to the treatment whereas measurement of a lower IL-6 protein level expression (or IL-6 concentration) is indicative that the patient is responder to the treatment and measurement of an equal IL-6 protein level (or IL-6 concentration) at the two or more times points indicates that Facioscapulohumeral muscular dystrophy does not progress (i.e.: is satble) in the patient.

Preferably, the concentration of IL-6 is measured in a serum sample or a plasma sample of said patient, most preferably a serum sample.

Said patient may be a man or a menopausal woman, preferably a man.

According to one aspect of the invention, the patient is assessed to be responder or non-responder based on at least two quantification of the concentration of IL-6 in a biological sample of a patient. For example, at least two quantifications may be performed: at least one quantification prior to the treatment and at least one quantification after the treatment. In another embodiment, at least two quantifications may be performed at various time points during and the treatment /or after completion of the treatment.

The term "responder" as used herein means a patient that demonstrates a positive treatment response to a Facioscapulohumeral muscular dystrophy treatment.

The term "non-responder" as used herein means a patient that does not demonstrate a positive treatment response to a Facioscapulohumeral muscular dystrophy treatment.

In one embodiment, the method of the invention thus may comprise the steps of
- (a) quantifying the concentration of IL-6 in a biological sample of a patient, and optionally comparing the obtained value with a reference control value and determining whether the obtained value is increased, decreased or is stable with regard to the reference control value,
- (b) providing a treatment to the patient, typically when said obtained value is increased with regard to the reference control value,
- (c) quantifying again the concentration of IL-6 in a biological sample of said patient and comparing the obtained concentration with the value prior to treatment and optionally to another corresponding control value or to the reference control value.

Typically the treatment is an inhibitor of the expression or the function of IL-6 (see below).

The inventors observed that IL-6 concentration was inversely correlated with MMT and directly correlated with CSS score, Vignos score and Brooke score. As such, a decreased IL-6 concentration of the patient after the treatment compared to prior to the treatment and optionally compared to a corresponding control value is relative to a responder patient and/or an effective treatment.

In a third aspect, the invention relates to the use as a biomarker, of the concentration of IL-6 in a biological sample. Preferably the biological sample is a serum sample or plasma sample, most preferably a serum sample.

The inventors showed that a higher concentration of IL-6 in the blood of the patient is positively correlated with the disease severity and the rate of disease progression. Given the known role of IL-6 and the existing treatments by anti-IL-6 in several inflammatory diseases, this molecule is not only a biomarker, but also a suitable therapeutic target.

In a fourth aspect, the invention relates to a method of treating a patient suffering from Facioscapulohumeral muscular dystrophy by providing the patient with (i) an inhibiting agent of the expression or the function of IL-6 such as Short interfering RNA (siRNA) or anti-IL-6 antibodies such as, for example, siltuximab, sirukumab, clazakizumab, elsilimomab, or olokizumab, preferably anti-IL-6 antibodies or (ii) with an inhibiting agent of the function of the IL-6 such as anti-CD126-antibodies (antibodies targeting the interleukin 6 receptor), for example tocilizumab, sarilumab or small molecules such as LMT-28 compound corresponding to formula

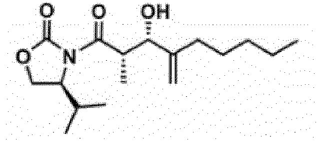

Thus the present invention also relates to an inhibitor of the expression or the function of IL-6 such as described above, for use in the treatment of a patient suffering from Facioscapulohumeral muscular dystrophy.

In one embodiment the method of the invention comprises steps of
- assessing the disease severity and/or the rate of disease progression in a patient suffering from Facioscapulohumeral muscular dystrophy as previously herein described, and
- treating said patient with an inhibitor of the expression or the function of IL-6 such as described above.

Said patient may be a man or a menopausal woman, preferably a man.

### Examples

### Patients and methods

### Enrollment and clinical assessment

All 100 patients were genetically confirmed for FSHD1, displaying a K4 D4Z4 copy number strictly inferior to 11 and an absence of concurrent mutations in the SMCHD1 gene. FSHD1 patients enrolled in a previous clinical study (Institutional Review Board reference: 13.045; National Agency for the Safety of Medicines and Health Products reference: 130942B-31 and National Commission on Informatics and Liberty Number: 1517317V0) came from different centers from France and Italy.

We excluded all patients:
- Presenting comorbidities or other criteria which could impact the immune response and as a consequence, the production of cytokines. More precisely, patients having been diagnosed for a severe and/or chronicle and/or recurrent pathology (autoimmune diseases, Parkinson, type II Diabetes, cancer), or having clinically significant organic disease.
- Vaccinated or, who resided in a tropical country during the last 3 months or, who had an infectious episode during the last three weeks prior to the blood draw.
- Being Human Immunodeficiency Virus (HIV) or BHV or CHV positive, having had a blood transfusion or Immunoglobulins Treatment during the last 3 months.
- Having been placed on a specific diet for medical reasons, consuming alcohol on a regular basis, having consumed recreative illicit drugs during the past 3 months.
- Being under immunosuppressive or immune-modulator treatments for more than 14 consecutive days during the last 6 months or during the last 2 weeks.
- Who have been participating to or were concurrently participating in another clinical study using investigational treatment in the last 3 months.

Patients were clinically assessed for disease severity estimating the MMT sum score, the Brooke score, the Vignos score and the CSS adjusted or not by age of clinical examination.

### Blood samples

Peripheral blood samples were obtained from fasting patients between 7:00 am and 9:00 am on workdays. Five milliliters of peripheral blood were drawn by venipuncture into serum Vacutainer tubes. For the serum collection, the blood was allowed to clot for 1 h before centrifugation (1500 x g, 10 min). The serum and plasma samples were stored in 0.5 ml aliquots at -80°C. For the measurements of cytokine and antibody levels, serum samples were thawed on ice, and 50 µl aliquots were prepared and stored at -80°C.

### Immunoassay

Serum levels of Granulocyte-Macrophage Colony-Stimulating Factor (GM-CSF), interferon (IFN)-gamma, interleukin(IL)-1alpha, IL-1beta, IL-2, IL-4, IL-5, IL-6, IL-7, IL-8, IL-10, IL-12p40, IL-12p70, IL-13, IL-15, IL-16, IL-17, Vascular Injury Growth Factor (VEGF) were measured using v-plex® (MSD) immunoassay using the commercially available Proinflammatory Panel 1 (MSD), and Cytokine Panel 1 (MSD). All assays were performed according to the manufacturer's instructions. The data were acquired on the v-plex® Sector Imager 2400 plate reader and analyzed using the Discovery Workbench 3.0 software (MSD). The standard curves for each cytokine were generated using the premixed lyophilized standards provided in the kits. Serial 2-fold dilutions of the standards were run to generate a 13-standard concentration set, and the diluent alone was used as a blank. The cytokine concentrations were determined from the standard curve using a robust-fit curve to transform the mean light intensities into concentrations. The lower limit of quantification (LLOQ) was determined for each cytokine and for each plate as twice the signal recorded for the blank.

### Statistical analysis

Outliers have been detected using GraphPad Prism7 ROUT algorithm with the more stringent parameter (Q=0.1%) (Motulsky and Brown, 2006). Heatmaps of all clinical and biomarkers data have been performed on Matlab software (R2017b) using Spearman rank correlation, and FDR have been computed using Benjamini and Hochberg method. Correlation plots and scatter plots have been done using GraphPad Prism 7. Biomarkers values have been transformed using LOG(y+1) and correlation computed using Spearman rank correlation.

### Results

The inventors assessed serum samples from 100 FSHD1 outpatients (Figure 1) for 20 soluble proteins: interferon (IFN)-gamma, Interleukin (IL)-1alpha, IL-1beta, IL-2, IL-4, IL-5, IL-6, IL-7, IL-8, IL-12p40, IL-12p70, IL-13, IL-17A, Tumor Necrosis Factor (TNF)-alpha and TNF-beta, Vascular Endothelial Growth Factor (VEGF)-A, Granulocyte Monocyte-Colony Stimulating Factor (GM-CSF). Out of these soluble proteins, IL-1-alpha, IL-1-beta, IL-2, IL-4, IL-5, IL-12p70 and IL-13 were below the level of detection in more than 20% of patients and were excluded from further analysis.

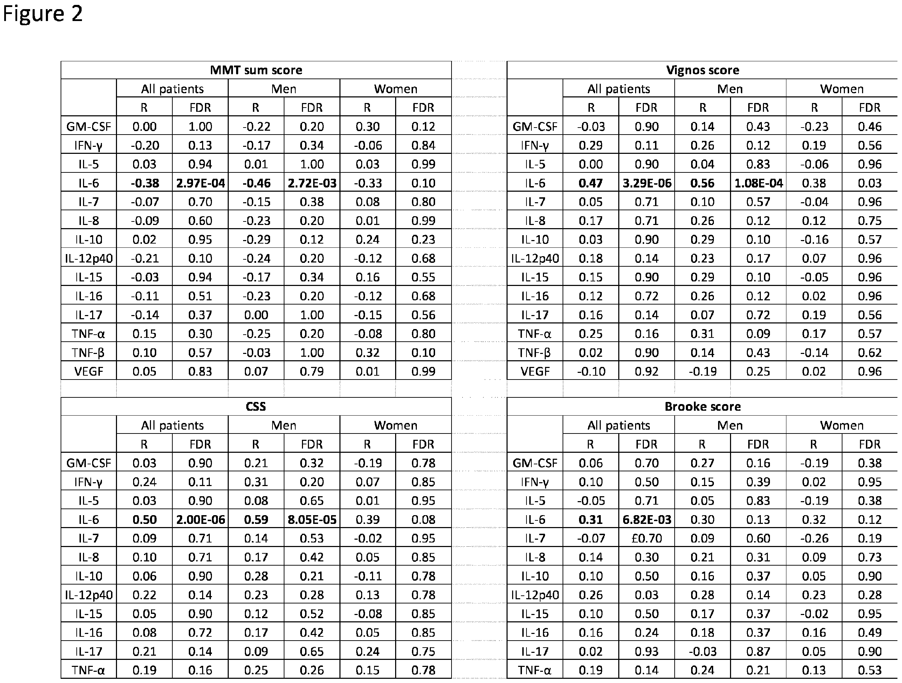

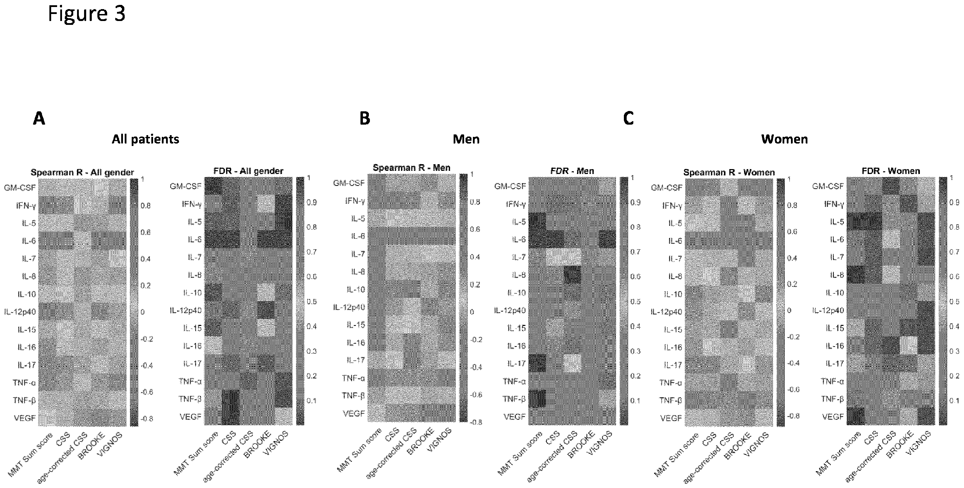

To investigate a possible association between serum cytokine levels and disease severity, the inventors computed for each soluble protein the Spearman's correlation coefficient between the MMT sum score, the CSS) adjusted or not by age at clinical assessment, the Brooke score and the Vignos score. After correction for multiple testing, the inventors found that serum levels of IL-6, but not those of any other tested biomarkers, correlated negatively with MMT (R = -0.38, FDR = 2.97 x 10⁻⁴) and positively with CSS (R = 0.55, FDR = 2.0 x 10⁻⁶), Brooke score (R = 0.31, FDR = 6.82 x 10⁻³) and Vignos score (R = 0.47, FDR = 3.29 x 10⁻⁶) (Figure 2, Figure 3).

Because sex-related disease modifiers affect sensitivity to FSHD1 (Teveroni et al., 2017), the inventors next studied the associations between serum cytokine levels and clinical scores in men and women respectively. While our results suggested that IL-6 was a biomarker of disease severity in both men and women, both the strength and the statistical significance of this association was higher in men than in women (Figure 2, Figure 3).

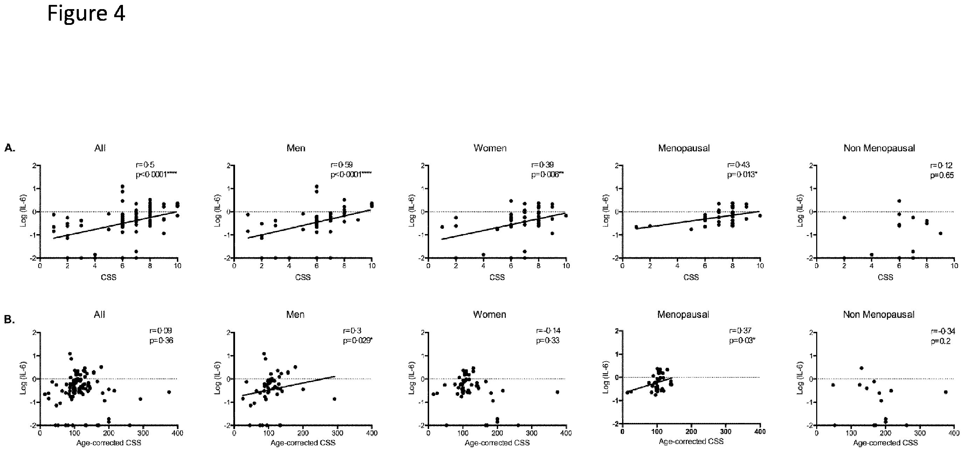

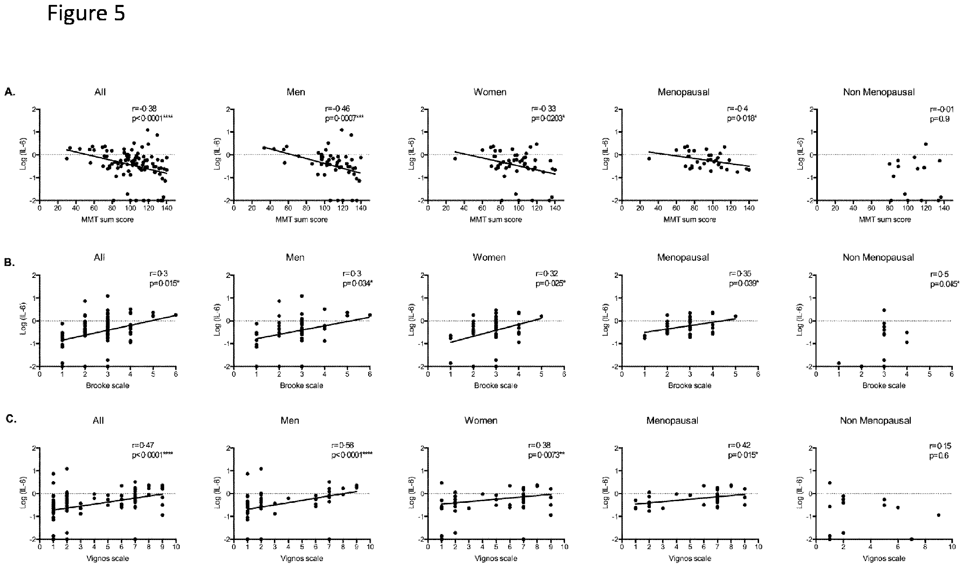

Because estrogens are disease modifiers that underlie sex-related differences in FSHD1, the inventors further studied the association between serum levels of IL-6 and clinical scores in menopausal and non-menopausal women (Figure 4, Figure 5). While serum levels of IL-6 correlated negatively with MMT sum score in all patients, men, women and menopausal women, they did not in non-menopausal women (Figure 5). Likewise, serum levels of IL-6 correlated positively with the CSS , the Brooke score and the Vignos score in all patients, men, women and menopausal women (Figure 4 and 5). In contrast, neither the MMT sum score nor the Vignos score correlated with serum levels of IL-6 in non-menopausal women. A low but statistically significant positive correlation was observed between serum levels of IL-6 and the Brooke score in non-menopausal women (Figure 5).

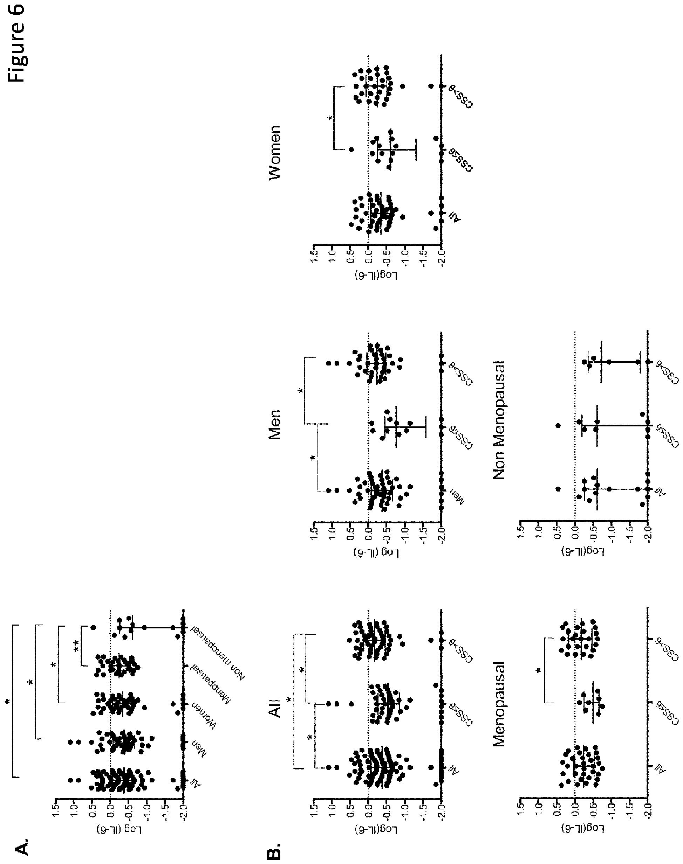

To further explore the associations between serum levels of IL-6 in individual subgroups of patients, the inventors stratified all patients, men, women, and menopausal and non-menopausal women into those with a CSS > 6 and those with a CSS ≤ 6. In men, women and menopausal women, but not non-menopausal women, patients with a CSS > 6 exhibited higher levels of IL-6 compared to those with a CSS ≤ 6 (Figure 6).

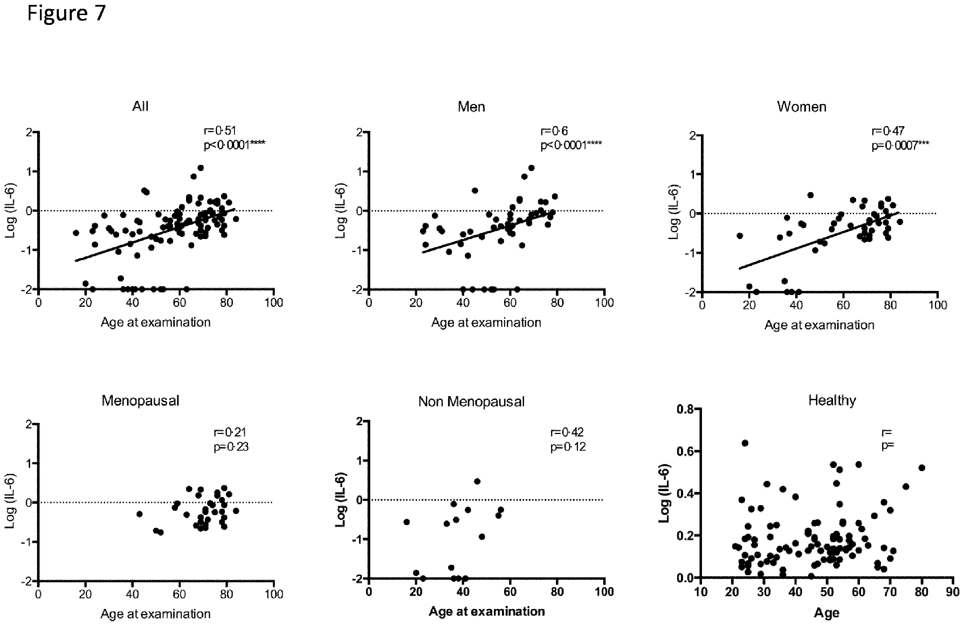

FSHD is a progressive disease and clinical symptoms aggravate with age. Therefore, the inventors further studied the association between serum levels of IL-6 and age at examination in all patients, men, women, menopausal and non-menopausal women, as well as in a cohort of healthy volunteers. Serum levels of IL-6 correlated positively with age in all patients, men and women (Figure 7). In contrast, no statistically significant correlation was observed between serum levels of IL-6 and age in menopausal women, non-menopausal women and healthy controls (Figure 7).

### References

Brooke, M.H., Griggs, R.C., Mendell, J.R., Fenichel, G.M., Shumate, J.B., and Pellegrino, R.J. (1981). Clinical trial in Duchenne dystrophy. I. The design of the protocol. Muscle Nerve 4, 186-197.
Florence, J.M., Pandya, S., King, W.M., Robison, J.D., Signore, L.C., Wentzell, M., and Province, M.A. (1984). Clinical trials in Duchenne dystrophy. Standardization and reliability of evaluation procedures. Phys Ther 64, 41-45.
Frisullo, G., Frusciante, R., Nociti, V., Tasca, G., Renna, R., Iorio, R., Patanella, A.K., Iannaccone, E., Marti, A., Rossi, M., et al. (2011). CD8(+) T cells in facioscapulohumeral muscular dystrophy patients with inflammatory features at muscle MRI. J Clin Immunol 31, 155-166.
Geng, L.N., Yao, Z., Snider, L., Fong, A.P., Cech, J.N., Young, J.M., van der Maarel, S.M., Ruzzo, W.L., Gentleman, R.C., Tawil, R., and Tapscott, S.J. (2012). DUX4 activates germline genes, retroelements, and immune mediators: implications for facioscapulohumeral dystrophy. Dev Cell 22, 38-51.
Kilmer, D.D., Abresch, R.T., McCrory, M.A., Carter, G.T., Fowler, W.M., Jr., Johnson, E.R., and McDonald, C.M. (1995). Profiles of neuromuscular diseases. Facioscapulohumeral muscular dystrophy. Am J Phys Med Rehabil 74, S131-139.
Lemmers, R.J., van der Vliet, P.J., Klooster, R., Sacconi, S., Camano, P., Dauwerse, J.G., Snider, L., Straasheijm, K.R., van Ommen, G.J., Padberg, G.W., et al. (2010). A unifying genetic model for facioscapulohumeral muscular dystrophy. Science 329, 1650-1653.
Motulsky, H.J., and Brown, R.E. (2006). Detecting outliers when fitting data with nonlinear regression - a new method based on robust nonlinear regression and the false discovery rate. BMC Bioinformatics 7, 123.
Olsen, D.B., Gideon, P., Jeppesen, T.D., and Vissing, J. (2006). Leg muscle involvement in facioscapulohumeral muscular dystrophy assessed by MRI. J Neurol 253, 1437-1441.
Ricci, E., Galluzzi, G., Deidda, G., Cacurri, S., Colantoni, L., Merico, B., Piazzo, N., Servidei, S., Vigneti, E., Pasceri, V., et al. (1999). Progress in the molecular diagnosis of facioscapulohumeral muscular dystrophy and correlation between the number of KpnI repeats at the 4q35 locus and clinical phenotype. Ann Neurol 45, 751-757.
Statland, J.M., Odrzywolski, K.J., Shah, B., Henderson, D., Fricke, A.F., van der Maarel, S.M., Tapscott, S.J., and Tawil, R. (2015). Immunohistochemical Characterization of Facioscapulohumeral Muscular Dystrophy Muscle Biopsies. J Neuromuscul Dis 2, 291-299.
Tasca, G., Monforte, M., Corbi, M., Granata, G., Lucchetti, D., Sgambato, A., and Ricci, E. (2018). Muscle Microdialysis to Investigate Inflammatory Biomarkers in Facioscapulohumeral Muscular Dystrophy. Mol Neurobiol 55, 2959-2966.
Tawil, R., McDermott, M.P., Pandya, S., King, W., Kissel, J., Mendell, J.R., and Griggs, R.C. (1997). A pilot trial of prednisone in facioscapulohumeral muscular dystrophy. FSH-DY Group. Neurology 48, 46-49.
Teveroni, E., Pellegrino, M., Sacconi, S., Calandra, P., Cascino, I., Farioli-Vecchioli, S., Puma, A., Garibaldi, M., Morosetti, R., Tasca, G., et al. (2017). Estrogens enhance myoblast differentiation in facioscapulohumeral muscular dystrophy by antagonizing DUX4 activity. J Clin Invest 127, 1531-1545.
Turki, A., Hayot, M., Carnac, G., Pillard, F., Passerieux, E., Bommart, S., Raynaud de Mauverger, E., Hugon, G., Pincemail, J., Pietri, S., et al. (2012). Functional muscle impairment in facioscapulohumeral muscular dystrophy is correlated with oxidative stress and mitochondrial dysfunction. Free Radic Biol Med 53, 1068-1079.
Vignos, P.J., Jr., Spencer, G.E., Jr., and Archibald, K.C. (1963). Management of progressive muscular dystrophy in childhood. JAMA 184, 89-96.

## Claims

1. An *in vitro* method for assessing the disease severity and/or the rate of disease progression in a patient suffering from Facioscapulohumeral muscular dystrophy, comprising the step of measuring the concentration of IL-6 in a biological sample of said patient and wherein the concentration of IL-6 is positively correlated with the severity and/or the rate of disease progression.

2. The *in vitro* method of claim 1 wherein said biological sample is a plasma sample or a serum sample of said patient, preferably a serum sample of said patient.

3. The *in vitro* method of any of the preceding claims wherein said patient is a man or a menopausal woman, preferably a man.

4. An *in vitro* method of monitoring a treatment efficacy in a patient suffering from Facioscapulohumeral muscular dystrophy receiving said treatment by using IL-6 as a biomarker.

5. The *in vitro* method of claim 4 comprising the step of measuring the concentration of IL-6 in a biological sample, preferably a plasma sample or a serum sample of said patient, preferably a serum sample of said patient.

6. The *in vitro* method of claim 4 or 5 wherein said patient is a man or a menopausal woman, preferably a man.

7. An inhibitor of the expression or the function of IL-6, preferably an anti-IL-6 antibody, for use in the treatment of a patient suffering from Facioscapulohumeral muscular dystrophy.

8. The method of claim 7 wherein said patient is a man or a menopausal woman, preferably a man.
